# EUROPÄISCHE PATENTSCHRIFT

(11) **EP 2 794 076 B1**
(45) Veröffentlichungstag und Bekanntmachung des Hinweises auf die Patenterteilung: **09.12.2015**
(21) Anmeldenummer: 12778961.8
(22) Anmeldetag: 17.10.2012
(51) Int. Cl.: B01D 63/08, B01D 65/00, B01D 65/02, A61L 2/02, B01D 25/127

(54) **FILTERHALTER UND VERFAHREN ZUR STERILISATION DES FILTERHALTERS**
FILTER HOLDER AND METHOD FOR STERILISING THE FILTER HOLDER
PORTE-FILTRE ET PROCÉDÉ POUR LE STÉRILISER

(30) Priorität: 19.12.2011 DE 102011121269
(43) Veröffentlichungstag der Anmeldung: 29.10.2014
(73) Patentinhaber: Sartorius Stedim Biotech GmbH, 37079 Göttingen (DE)
(72) Erfinder: AJAM, Mohammed Saeed, 44227 Dortmund (DE); MATTERN, Jonas, 34212 Melsungen (DE); SCHÄFER, Jan, 34295 Edermünde (DE)
(74) Vertreter: Schneider, Peter Christian
(86) Internationale Anmeldenummer: PCT/EP2012/004337
(87) Internationale Veröffentlichungsnummer: WO 2013/091747

(56) Entgegenhaltungen:
- DE-A1- 3 708 734
- DE-C2- 3 708 733
- US-A- 4 846 970

## Beschreibung

### Gebiet der Erfindung

Die Erfindung betrifft einen Filterhalter zur Aufnahme von Filterkassetten mit zwei relativ zu einander auf mindestens einer Schiene verschiebbaren Platten, zwischen die die Filterkassetten einspannbar sind, und mit einem Antrieb, wobei eine erste Platte fest und eine zweite parallele Platte über den Antrieb mit einem vorgebbaren Spanndruck verschiebbar angeordnet ist, und wobei der Antrieb von einem mit Druck beaufschlagbarem Hydraulikzylinder gebildet wird, über dessen verschieblichen Kolben die zweite Platte mit dem vorgegebenen Spanndruck gegen die Filterkassetten spannbar ist.

Die Erfindung betrifft weiterhin ein Verfahren zur Sterilisation eines Filterhalters in einem Autoklaven mit Filterkassetten, die zwischen zwei relativ zu einander auf mindestens einer Schiene verschiebbaren Platten eingespannt werden, wobei für die Sterilisation die zweite verschiebbare Platte über mindestens eine parallel zur Schiene angeordnete Zugstange mit einem Federspannelement mit einem vorgegebenen Zugstangendruck gegen die fest mit der Schiene verbundene erste Platte gespannt und der Filterhalter in dem Autoklaven autoklaviert wird.

### Stand der Technik

Aus dem Firmenprospekt "SARTOFLOW® 10 Stainless Steel Holder" ist ein Filterhalter zur Aufnahme von Filterkassetten mit zwei relativ zueinander auf parallelen Schienen verschiebbaren Platten, zwischen die die Filterkassetten einspannbar sind, bekannt. Dabei ist eine erste Platte fest und eine zweite parallele Platte über einen hydraulischen Antrieb mit einem vorgebbaren Spanndruck verschiebbar angeordnet. Der hydraulische Antrieb ist als ein Hydraulikzylinder ausgebildet, der an einer dritten fest mit den Schienen verbundenen Platte angeordnet ist und einen verschieblichen Kolben aufweist, der mit einem vorgebbaren Spanndruck die zweite verschiebbare Platte in Richtung der ersten Platte verschiebt.

Durch die Verwendung eines hydraulischen Antriebs lässt sich die zweite Platte mit hoher Genauigkeit verschieben und damit die vorgesehene Einspannkraft zum Verschieben der zweiten Platte und damit der Anpressdruck der Filterkassetten genau festlegen.

Nachteilig bei einem derartigen Filterhalter, der sich grundsätzlich bewährt hat, ist, dass der Filterhalter nicht mit den eingespannten Filterkassetten autoklaviert werden kann. Aufgrund der Hydraulikflüssigkeit des hydraulischen Antriebs würde eine unerwünschte Ausdehnung im Hydraulikzylinder erfolgen, die zu hohen Anpressdrücken bzw. Kräften führen würde.

Auch aus der DE 37 08 734 A1 ist ein Filterhalter zur Aufnahme von Filterkassetten mit zwei relativ zueinander auf parallelen Schienen verschiebbaren Platten, zwischen die die Filterkassetten einspannbar sind, bekannt. Dabei ist eine erste Platte fest und eine zweite parallele Platte über einen hydraulischen Antrieb mit einem vorgebbaren Spanndruck verschiebbar angeordnet. Der hydraulische Antrieb ist als ein Hydraulikzylinder ausgebildet, der an einer dritten fest mit den Schienen verbundenen Platte angeordnet ist und einen verschieblichen Kolben aufweist, der mit einem vorgebbaren Spanndruck die zweite verschiebbare Platte in Richtung der ersten Platte verschiebt.

Dabei ist es möglich den Filterhalter im In-Line-Betrieb mit Heißdampf zu sterilisieren. Um eine Zerstörung der Filterkassetten bei Beaufschlagung des Filterhalters mit Heißdampf zu verhindern, muss während der Beschickung mit Heißdampf durch eine relativ aufwendige Regelung der Spanndruck der verschiebbaren Platten auf die Filterkassetten geregelt werden. Nachteilig ist auch hier, dass der Filterhalter nicht einfach mit den eingespannten Filterkassetten in einem Autoklaven autoklaviert werden kann.

Weiterhin ist aus der DE 37 08 733 C2 ein Filterhalter zur Aufnahme von Filterkassetten mit zwei relativ zueinander auf einer Schiene verschiebbaren Platten, zwischen die die Filterkassetten einspannbar sind, bekannt. Dabei sind eine erste Platte und eine zweite parallele Platte verschiebbar angeordnet. Parallel zu der Schiene ist eine Zugstange mit einem Federspannelement vorgesehen, über die die beiden Platten miteinander verbindbar und mit einem vorgegebenen Zugstangendruck gegen die Filterkassetten spannbar sind. Die Platten mit den dazwischen liegenden Filterkassetten werden manuell über die Zugstange miteinander verschraubt. Für den Filtrationsprozess werden die Federspannelemente blockiert und der Spanndruck, mit der die bewegliche Platte in Richtung der festen Platte gezogen wird, wird manuell über einen Drehmomentschlüssel eingestellt. Zur Verringerung des Spanndrucks wird durch eine Drehung der Zugstange die Blockierung des Federspannelementes aufgehoben, so dass beim Sterilisieren mit Heißdampf ein Ausgleich über das Federspannelement erfolgen kann. Dabei steht dem Vorteil des Heißdampfsterilisierens eine gewisse Ungenauigkeit bei der Einstellung des Spanndrucks während des Filtrationsprozesses gegenüber.

Aus der US 4 846 970 A ist eine Testvorrichtung für Membranfilter bekannt, die einen Filterhalter zur Aufnahme von Membranfiltern zwischen zwei Verteilerplatten aufweist, bekannt. Dabei ist über einen Hydraulikantrieb eine Druckplatte gegen die Verteilerplatten spannbar. Eine Zugstangenanordnung mit Feder zieht die Druckplatte von den Verteilerplatten weg. Eine Entfernung des Hydraulikzylinders ist bei dieser bekannten Vorrichtung nicht möglich und zudem fällt ohne Hydraulikdruck die Verspannung der Verteilerplatten mit dem dazwischen angeordneten Membranfilter weg.

### Aufgabenstellung

Aufgabe der vorliegenden Erfindung ist es daher, die bekannten Filterhalter so zu verbessern, dass einerseits der Druck zum Verspannen der Platten mit den dazwischenliegenden Filterkassetten mit hoher Genauigkeit über einen Antrieb eingestellt werden kann und dass andererseits der Filterhalter mit eingespannten Filterkassetten autoklaviert werden kann.

Weitere Aufgabe ist es, ein entsprechendes Verfahren zum Sterilisieren des Filterhalters mit Filterkassetten anzugeben.

### Darstellung der Erfindung

Die Aufgabe bezüglich des Filterhalters wird in Verbindung mit dem Oberbegriff des Anspruches 1 dadurch gelöst, dass der Antrieb abnehmbar ausgebildet ist, dass parallel zu der mindestens einen Schiene mindestens eine Zugstange mit einem Federspannelement vorgesehen ist, über die die beiden Platten miteinander verbindbar und mit einem vorgebbaren Zugstangendruck gegen die Filterkassetten spannbar sind.

Durch die Verwendung eines Antriebes zum Verschieben der zweiten Platte lässt sich der Spanndruck, mit dem die Filterkassetten im Filterhalter verspannt werden, vorgeben und präzise einhalten. Dies ist für die Durchführung eines Filtrationsprozesses wichtig. Durch die zusätzliche Anordnung einer Zugstange mit einem Federspannelement kann ein vorgebbarer Zugstangendrück eingestellt werden, der durch das Federspannelement in gewissen Grenzen variabel ist, wodurch eine Druckerhöhung durch die aus der Sterilisation mit Heißdampf resultierende Ausdehnung des Filterhalters und eine damit verbundene Beschädigung der Filterkassetten verhindert wird. Insbesondere bei Verwendung eins Hydraulikzylinders als Antrieb würde ein Autoklavieren des Hydraulikzylinders zu einer unerwünschten Druckerhöhung infolge der Ausdehnung von Hydraulikflüssigkeit im Zylinder führen. Nach dem Verspannen der Filterkassetten über die Zugstange mit Federspannelement kann die Verspannung über den Antrieb aufgehoben und der nicht autoklavierbare Antrieb entfernt werden.

Dadurch ist es möglich, den Filterhalter mit den verspannten Kassetten in einem Autoklaven bzw. mit Heißdampf zu sterilisieren. Der erfindungsgemäße Filterhalter ermöglicht es somit, dampfsterilisierbare Filterkassetten im Autoklaven zu sterilisieren und aseptisch mit einem bereits sterilen "single-use-Paket" zu verbinden. Unter single-use-Paket soll hier beispielsweise ein System bestehend aus einem Rezirkulationsbehälter und einem Filtratbehälter mit entsprechenden Schlauchverbindungen verstanden werden, die mit dem Filterhalter mit Filterkassetten zu einem Gesamtsystem verbunden werden. Dabei können sämtliche Teile des Gesamtsystems mit Ausnahme des Filterhalters ohne Filterkassetten als Einwegteile bzw. single-use-Teile ausgebildet sein. Der Filterhalter mit den Filterkassetten kann zudem nach einem Filtrationsprozess erneut autoklaviert werden und damit zur Detoxifizierung eingesetzt werden.

Der Hydraulikzylinder kann dabei über eine Hydraulikleitung mit einer Pumpe verbunden sein, wobei zwischen Pumpe und dem Hydraulikzylinder bevorzugt ein Manometer angeordnet ist. Die Pumpe kann dabei insbesondere auch als eine Handpumpe ausgebildet sein.

Gemäß einer bevorzugten Ausführungsform der Erfindung ist der zweiten Platte auf ihrer der ersten Platte abgewandten Seite eine dritte Platte vorgelagert, die fest mit der mindestens einen Schiene verbunden ist, wobei der abnehmbare Antrieb an der dritten Platte fixierbar ist. An der dritten Platte lässt sich der Antrieb leicht montieren und demontieren, beispielsweise über eine Verriegelungsvorrichtung oder eine Verschraubung.

Gemäß einer weiteren bevorzugten Ausführungsform der Erfindung sind vier Schienen vorgesehen, auf denen die zweite Platte gegenüber der ersten Platte verschiebbar ist. Insbesondere durch die Verwendung von drei oder vier Schienen wird eine Parallelität zwischen den Platten sichergestellt, die für eine gleichmäßige Druckausübung auf die zwischen ihnen zu verspannenden Filterkassetten notwendig ist.

Nach einer weiteren bevorzugten Ausführungsform der Erfindung sind zwei Zugstangen mit je einem Federspannelement vorgesehen, über die die beiden Platten miteinander verbindbar und mit der vorgebbaren Zugkraft gegen die Filterkassetten spannbar sind. Dabei ist der Filterhalter ohne Antrieb mit den Filterkassetten, die über die Zugstangen mit Federspannelement vorgespannt sind, autoklavierbar.

Die Aufgabe bezüglich des Verfahrens wird in Verbindung mit dem Oberbegriff des Anspruches 7 dadurch gelöst, dass nach dem Autoklavieren für den Filtrationsprozess ein Antrieb ortsfest mit der Schiene verbunden wird, dass der Antrieb die zweite Platte mit einem vorgegebenen Spanndruck gegen die erste Platte verspannt und dass die Zugstange mit dem Federspannelement entlastet wird.

Durch die Verwendung einer Zugstange mit Federspannelement kann der Filterhalter mit den eingespannten Filterkassetten in einem Autoklaven mit Heißdampf sterilisiert werden. Nach dem Autoklavieren kann ein Antrieb ortsfest mit der Schiene verbunden werden, der die zweite Platte mit einer vorgegeben Kraft bzw. Spanndruck gegen die erste Platte mit relativ hoher Genauigkeit verspannt. Anschließend kann die Zugstange mit dem Federspannelement entlastet werden.

Damit kann zum einen der Filterhalter mit Kassetten autoklaviert und zum anderen für den Filtrationsprozess präzise mit einer vorgebbaren Kraft bzw. Druck verspannt werden. Dadurch, dass die Zugstange mit dem Federspannelement erst entlastet wird, wenn auf die zweite Platte über den Antrieb Spanndruck ausgeübt wird, werden Leckagen zwischen den Prozessschritten vermieden.

Gemäß einer bevorzugten Ausführungsform der Erfindung ist der Zugstangendruck zum Verspannen der Platten für die Sterilisation geringer als der Spanndruck zum Verspannen der Platten während des Filtrationsvorganges. Dadurch wird sichergestellt, dass beim Autoklavieren der Druck auf die Platten in einem vorgesehenen Bereich bleibt und die Filterkassetten während des Autoklaviervorganges relativ schonend abgedichtet werden.

Gemäß einer weiteren bevorzugten Ausführungsform der Erfindung weist der Antrieb einen Hydraulikzylinder auf, der so an eine der zweiten Platte vorgelagerte und mit der Schiene verbundene dritte Platte aufgesteckt bzw. an ihr fixiert wird, dass sein verschieblicher Kolben den notwendigen Spanndruck auf die zweite Platte ausübt und die Filterkassetten verspannt. Der Hydraulikzylinder des Antriebs lässt sich: leicht auf die dritte Platte aufstecken und mit dieser sicher verbinden, wobei sein verschieblicher Kolben die notwendige Kraft auf die zweite Platte zur Vorspannung der Filterkassetten ausüben kann.

Nach einer weiteren bevorzugten Ausführungsform der Erfindung wird zum Autoklavieren der Antrieb aufgesteckt und eine dem Zugstangendruck entsprechender reduzierter Spanndruck auf die zweite Platte ausgeübt, wobei anschließend die Zugstange angezogen und die Platten über das Federspannelement mit dem vorgesehenen Zugstangendruck verspannt werden. Danach wird der Antrieb entspannt und abgenommen, wobei nach dem Autoklavieren der Antrieb wieder aufgesteckt und der für den Filtrationsprozess vorgesehene Spanndruck eingestellt wird und wobei anschließend die Zugstange entlastet wird. Bei einem derartigen Vorgehen wird sichergestellt, dass die Filterkassetten stets und gerade zwischen den Prozessschritten ausreichend verspannt sind.

Nach einer weiteren bevorzugten Ausführungsform der Erfindung wird der Filterhalter mit einem an den Filterkassetten angeschlossenen Schlauchset autoklaviert und anschließend aseptisch in einen bereits sterilen Prozessablauf eingebunden.

Weitere Einzelheiten der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibung der Zeichnungen

In den Zeichnungen zeigen:
- Figur 1:: eine Seitenansicht eines Filterhalters teilweise im Schnitt mit gestrichelt angedeutetem Filterkassettenstapel, der von Adapterplatten eingegrenzt ist;
- Figur 2:: eine Draufsicht auf den Filterhalter von Figur 1 aus Richtung II;
- Figur 3:: eine Seitenansicht eines weiteren Filterhalters teilweise im Schnitt mit gestrichelt angedeutetem Filterkassettenstapel, der von Adapterplatten eingegrenzt ist;
- Figur 4:: eine Draufsicht auf den Filterhälter von Figur 3 aus Richtung IV;
- Figur 5:: eine Seitenansicht auf den Filterhalter von Figur 1 aus Richtung V;
- Figur 6:: eine Seitenansicht auf den Filterhalter von Figur 3 aus Richtung VI und

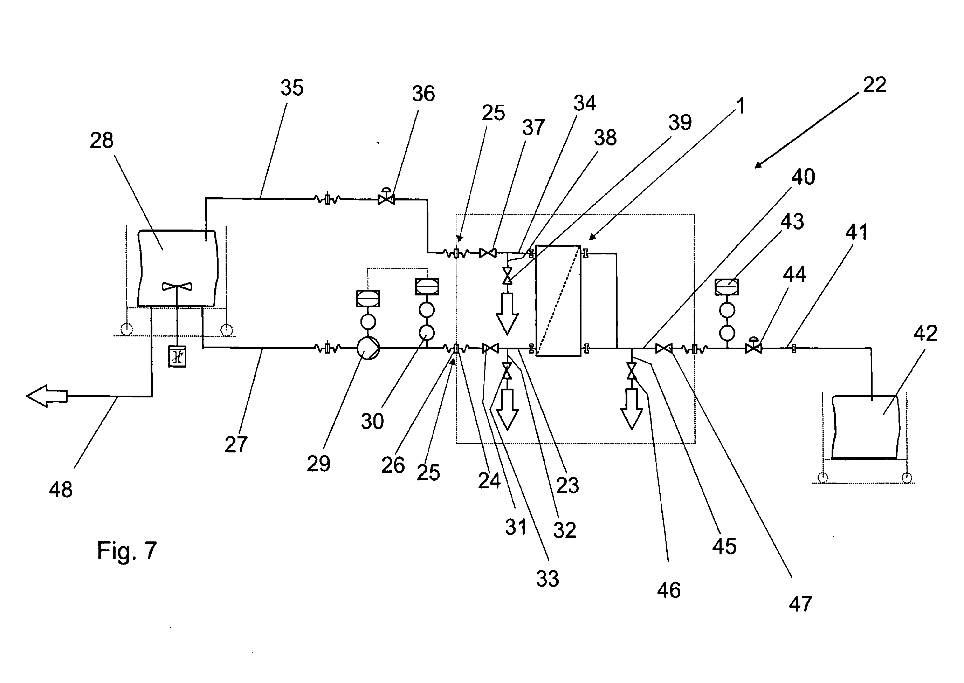

- Figur 7:: eine schematische Darstellung eines Gesamtsystems mit angeschlossenem Filterkassettenstapel.

### Beschreibung der Ausführungsbeispiele

Ein Filterhalter 1 besteht im Wesentlichen aus Schienen 2, Platten 3, 4, Zugstangen 5, Federspannelementen 6 und einem Antrieb 7.

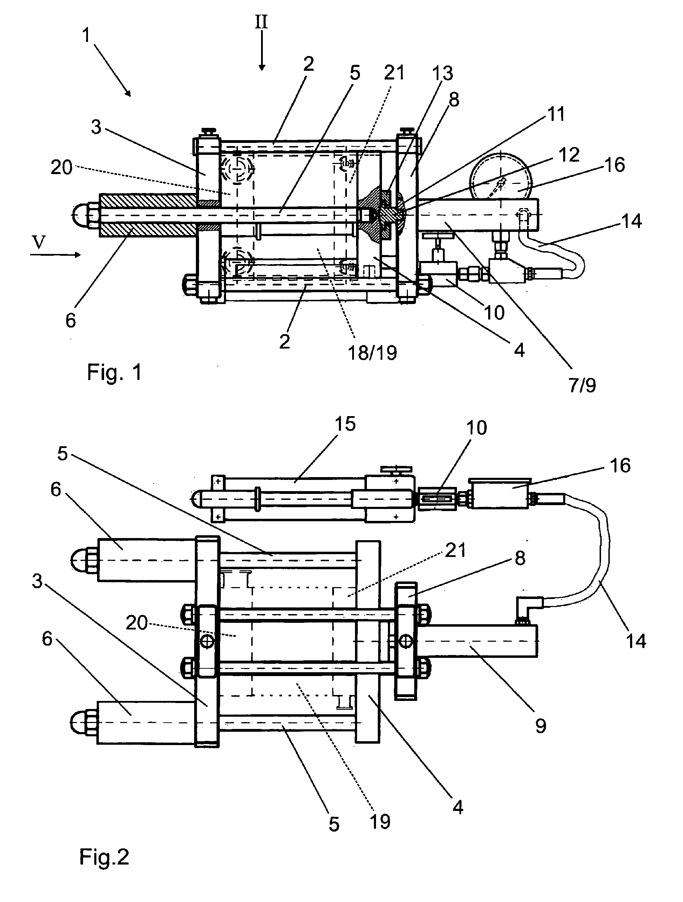

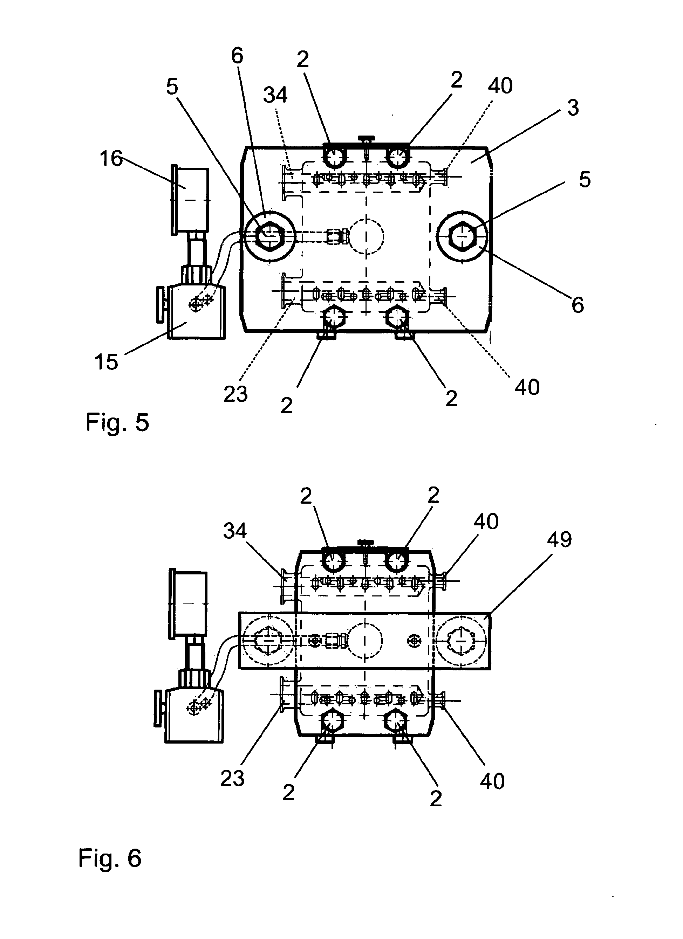

Entsprechend dem Ausführungsbeispiel der Figuren 1,2 und 5 sind vier Schienen 2 mit der ersten Platte 3 fest verbunden. Die zweite Platte 4 ist auf den Schienen 2 jeweils verschieblich geführt. Der zweiten Platte 4 ist auf ihrer der ersten Platte 3 abgewandten Seite eine dritte Platte 8 vorgelagert, die ebenfalls mit den Schienen 2 fest verbunden ist. An der dritten Platte 8 ist der Antrieb 7 abnehmbar angeordnet. Der Antrieb 7 wird von einem mit Druck beaufschlagbarem Hydraulikzylinder 9 gebildet, der ein Außengewinde aufweist mit dem er in eine Öffnung der dritten Platte 8, die ein Innengewinde aufweist, einschraubbar und damit fixierbar ist. Der Hydraulikzylinder 9 weist einen verschieblichen Kolben 11 auf, der über ein Druckstück 12 einen Spanndruck auf die verschiebliche zweite Platte 4 ausübt. Das Druckstück 12 ist über einen Ring 13 mit der zweiten Platte 4 verbunden. Der Hydraulikzylinder 9 ist über eine Hydraulikleitung 14 mit einer Pumpe 15, die in den Ausführungsbeispielen als eine Handpumpe ausgebildet ist, verbunden. Zwischen Pumpe 15 und dem Hydraulikzylinder 9 ist ein Manometer 16 zur Anzeige des Druckes im Hydraulikzylinder 9 angeordnet. Zwischen Pumpe 15 und Manometer 16 kann ein Nadelventil 10 angeordnet sein.

Die erste Platte 3 und die zweite Platte 4 sind zusätzlich über zwei Zugstangen 5 miteinander verbunden. Dabei weist jede der Zugstangen 5 ein Federspannelement 6 auf. Über die Zugstangen 5 sind die Platten 3, 4 mit einem durch nicht dargestellte Druckfedern vorgegebenen Zugstangendruck der Federspannelemente 6 gegeneinander verspannbar.

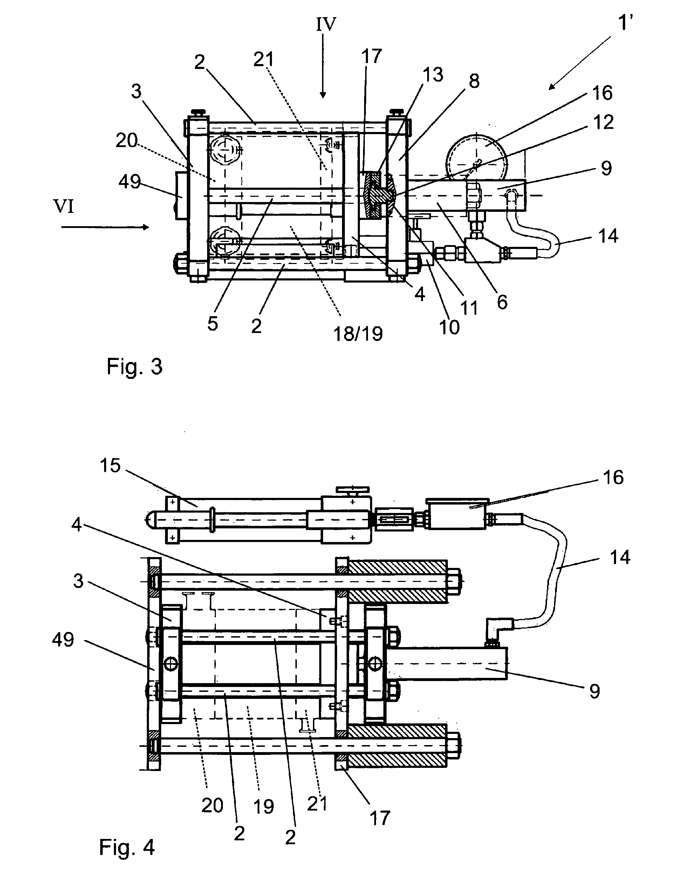

Entsprechend: dem Ausführungsbeispiel der Figuren 1, 2 und 5 sind die Zugstangen 5 mit ihren Federspannelementen 6 der ersten Platte 3 vorgelagert. Entsprechend den Ausführungsbeispielen der Figuren 3, 4 und 6 sind die Zugstangen 5 mit ihren Federspannelementen 6 der verschiebbaren zweiten Platte 4 über einen ersten Querhalter 17, der mit der zweiten Platte 4 verschraubbar ist, vorgelagert. Ein zweiter Querhalter 49 ist mit der ersten Platte 3 verschraubt und nimmt die Enden der Zugstangen 5 auf.

Zwischen die Platten 3, 4 ist ein Kassettenstapel 18 einspannbar, der eine Mehrzahl von Filterkassetten 19 und an beiden den Platten 3, 4 zugewandten Enden jeweils eine Filterplatte: 20, 21 aufweist, wobei die der ersten Platte 3 benachbarte erste Filterplatte 20 als eine Feedplatte/Retentatplatte, die die zu filtrierende Flüssigkeit verteilt, und die zweite Filterplatte 21 als eine Permeatplatte ausgebildet ist, die das Permeat zusammenführt.

Über den Antrieb 7 lassen sich die Filterkassetten 19 und die Filterplatten 20, 21 mit einem vorgebbaren Spanndruck zwischen den Platten 3, 4 verspannen. Gleichzeitig oder allein ist eine Verspannung der Filterkassetten 19 und der Filterplatten 20, 21 über die Zugstangen 5 und Federspannelemente 6 mit einem vorgebbaren Zugstangendruck möglich. Der Zugstangendruck ist dabei gegenüber dem Spanndruck reduziert.

Der Filterhalter 1, der mit seinem Kassettenstapel 18 in einem Autoklaven sterilisierbar ist, kann aseptisch in ein steriles Gesamtsystem 22, das als ein Einweg(single-use)-System ausgebildet ist, eingefügt werden. Zu diesem Zweck weist die erste Filterplatte 20 eine Zulaufleitung 23 mit einem ersten Teil 24 eines Sterilverbinders 25, der mit einem zweiten Teil 26 des Sterilverbinders 25 verbindbar ist. Das zweite Teil 26 ist dabei an einer Ablaufleitung 27 eines Rezirkulationsbehälters 28 angeordnet. Weiterhin ist in der Ablaufleitung 27 eine Rezirkulationspumpe 29 angeordnet, der zum Filterhalter 1 hin ein Druckmesser 30 zur Bestimmung des Speisedrucks nachgelagert ist. Über den Druckmesser 30 kann die Rezirkulationspumpe 29 gesteuert werden. In der Zulaufleitung 23 des Filterhalters 1 bzw. des Kassettenstapels 18 ist ein Ventil 31 angeordnet. Für die Autoklavierung weist die Zulaufleitung 23 eine Verbindungsleitung 32 zu einem nicht dargestellten Becherglas auf, die von einem Ventil 33 verschließbar ist.

Die erste Filterplatte 20 weist eine Retentatleitung 34 auf, die ebenfalls mit einem Sterilverbinder 25 mit einer Rezirkulationsleitung 35 die erste Filterplatte 20 mit dem Rezirkulationsbehälter 28 verbindet, so dass Retentat aus dem Kassettenstapel 18 in den Rezirkulationsbehälter 28 zurückgeführt werden kann. In der Rezirkulationsleitung 35 ist in an sich bekannter Weise ein Regelventil 36 angeordnet. Zwischen der erste Filterplatte 20 und dem Sterilverbinder 25 ist in der Retentatleitung 35 ein Ventil 37 angeordnet. Auch die Rezirkulationsleitung 35 weist eine Verbindungsleitung 38 auf, die für die Autoklavierung ebenfalls über ein Ventil 39 mit einem Becherglas verbindbar ist.

Die zweite Filterplatte 21 des Kassettenstapels 18 weist eine Permeatleitung 40 auf, die über einen Sterilverbinder 25 mit einer Zuleitung 41 eines Filtrat- bzw. Permeatbehälters 42 verbunden ist. In der Zuleitung 41 zum Permeatbehälter 42 ist ein Drucksensor 43 und ein Regelventil 44 angeordnet.

Die Permeatleitung 40 weist für die Autoklavierung eine Verbindungsleitung 45 zu einem Becherglas auf, die über ein Ventil 46 verschließbar ist. In der Permeatleitung 40 ist dem Sterilverbinder 25 ein Ventil 47 vorgelagert.

Der Rezirkulationsbehälter 28 weist eine zweite Ablaufleitung 48 zu einem nicht dargestellten Erntebehälter hin auf.

Zum Autoklavieren des Filterhalters 1 wird der Antrieb 7, d.h. der Hydraulikzylinder 9, durch Aufstecken mit der dritten Platte 8 verbunden und beispielsweise über ein Außengewinde fixiert. Der Kassettenstapel 18 wird mit den Filterkassetten 19 und den beiden Filterplatten 20, 21 zwischen die Platten 3, 4 eingesetzt, wobei die zweite Platte 4 durch den Kolben 11 des Hydraulikzylinders mit einem dem Zugstangendruck entsprechenden reduzierten Spanndruck in Richtung der ersten Platte 3 verschoben wird, so dass der Kassettenstapel 18 mit dem reduzierten Spanndruck verspannt wird. Danach werden die Zugstangen angezogen und die Platten 3, 4 über die Federspannelemente 6 mit dem vorgesehenen Zugstangendruck verspannt. Anschließend wird der Kolben 11 zurückgezogen und der Hydraulikzylinder 9 mit dem Kolben 11 von der dritten Platte 8 abgenommen und der Filterhalter 1 in einem nicht dargestellten Autoklaven mit Heißdampf sterilisiert. Nach dem Sterilisieren bzw. Autoklavieren wird der Hydraulikzylinder 9 wieder auf die dritte Platte 8 aufgesteckt und der für den Filtrationsprozess vorgesehene Spanndruck wird eingestellt, wobei anschließend die Zugstangen 5 wieder entlastet werden. Nunmehr kann der autoklavierte Filterhalter mit dem Kassettenstapel 18 über die Sterilverbinder 25 in das Gesamtsystem 22 eingefügt werden.

Natürlich stellen die in der speziellen Beschreibung diskutieren und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben.

### Bezugszeichenliste

- 1: Filterhalter
- 2: Schienen
- 3: erste Platte
- 4: zweite Platte
- 5: Zugstangen
- 6: Federspannelemente
- 7: Antrieb
- 8: dritte Platte
- 9: Hydraulikzylinder
- 10: Nadelventil
- 11: Kolben von 9
- 12: Druckstück
- 13: Ring
- 14: Hydraulikleitung
- 15: Pumpe
- 16: Manometer
- 17: Querhalter
- 18: Kassettenstapel
- 19: Filterkassetten
- 20: Filterplatte
- 21: Filterplatte
- 22: Gesamtsystem
- 23: Zulaufleitung von 20
- 24: erstes Teil von 5
- 25: Sterilverbinder
- 26: zweites Teil von 5
- 27: Ablaufleitung
- 28: Rezirkulationsbehälter
- 29: Rezirkulationspumpe
- 30: Druckmesser
- 31: Ventil
- 32: Verbindungsleitung von 23
- 33: Ventil
- 34: Retentatleitung
- 35: Rezirkulationsleitung
- 36: Regelventil
- 37: Ventil von 34
- 38: Verbindungsleitung von 34
- 39: Ventil von 38
- 40: Permeatleitung
- 41: Zuleitung von 42
- 42: Permeatbehälter
- 43: Drucksensor
- 44: Regelventil
- 45: Verbindungsleitung
- 46: Ventil von 45
- 47: Ventil von 40
- 48: zweite Ablaufleitung von 28
- 49: zweiter Querhalter von 3

## Patentansprüche

1. Filterhalter (1) zur Aufnahme von Filterkassetten (19) mit zwei relativ zu einander auf mindestens einer Schiene (2) verschiebbaren Platten (3, 4), zwischen die die Filterkassetten (19) einspannbar sind, und mit einem Antrieb (7), wobei eine erste Platte (3) fest und eine zweite parallele Platte (4) über den Antrieb (7) mit einem vorgebbaren Spanndruck verschiebbar angeordnet ist, und wobei der Antrieb (7) von einem mit Druck beaufschlagbarem Hydraulikzylinder (9) gebildet, wird, über dessen verschieblichen Kolben (11) die zweite Platte (4) mit dem vorgegebenen Spanndruck gegen die Filterkassetten (19) spannbar ist,
**dadurch gekennzeichnet,**
**dass** der Antrieb (7) abnehmbar ausgebildet ist,
**dass** parallel zu der mindestens einen Schiene (2) mindestens eine Zugstange (5) mit einem Federspannelement (6) vorgesehen ist, über die die beiden Platten (3, 4) miteinander verbindbar und mit einem vorgebbaren Zugstangendruck gegen die Filterkassetten (19) spannbar sind.

2. Filterhalter nach Anspruch 1,
**dadurch gekennzeichnet,**
**dass** der zweiten Platte (4) auf ihrer der ersten Platte (3) abgewandten Seite eine dritte fest mit der mindestens einen Schiene (2) verbundene Platte (8) vorgelagert ist, und
**dass** der abnehmbare Antrieb (7) an der dritten Platte (8) fixierbar ist.

3. Filterhälter nach Anspruch 1 oder 2,
**dadurch gekennzeichnet,**
**dass** der Hydraulikzylinder (9) über eine Hydraulikleitung (14) mit einer Pumpe (15) verbunden ist und
**dass** zwischen der Pumpe (15) und dem Hydraulikzylinder (9) ein Manometer (16) angeordnet ist.

4. Filterhalter nach einem der Ansprüche 1 bis 3,
**dadurch gekennzeichnet,**
**dass** vier Schienen (2) vorgesehen sind auf denen die zweite Platte (4) gegenüber der ersten Platte (3) verschiebbar ist.

5. Filterhalter nach einem der Ansprüche 1 bis 4,
**dadurch gekennzeichnet,**
**dass** zwei Zugstangen (5) mit je einem Federspannelement (6) vorgesehen sind über die die beiden Platten (3, 4) miteinander verbindbar und mit dem vorgebbaren Zugstangendruck gegen die Filterkassetten (19) spannbar sind.

6. Filterhalter nach einem der Ansprüche 1 bis 5,
**dadurch gekennzeichnet,**
**dass** der Filterhalter (1) ohne Antrieb (7) mit den Filterkassetten (19), die über die mindestens eine Zugstange (5) mit Federspannelement (6) vorgespannt sind, autoklavierbar ist.

7. Verfahren zur Sterilisation eines Filterhalters (1) in einem Autoklaven mit Filterkassetten (19), die zwischen zwei relativ zu einander auf mindestens einer Schiene (2) verschiebbaren Platten (3, 4) eingespannt werden, wobei für die Sterilisation die zweite verschiebbare Platte (4) über mindestens eine parallel zur Schiene (2) angeordnete Zugstange (5) mit einem Federspannelement (6) mit einem vorgegebenen Zugstangendruck gegen die fest mit der Schiene verbundene erste Platte (3) gespannt und der Filterhalter (1) in dem Autoklaven autoklaviert wird, **dadurch gekennzeichnet,**
**dass** nach dem Autoklavieren für den Filtrationsprozess ein Antrieb (7) ortsfest mit der Schiene (2) verbunden wird,
**dass** der Antrieb (7) die zweite Platte (4) mit einem vorgegebenen Spanndruck gegen die erste Platte (3) verspannt, und
**dass** die Zugstange (5) mit dem Federspannelement (6) entlastet wird.

8. Verfahren nach Anspruche 7,
**dadurch gekennzeichnet,**
**dass** der Zugstangendruck zum Verspannen der Platten (3, 4) für die Sterilisation geringer ist als der Spanndruck zum Verspannen der Platten (3, 4) während des Filtrationsvorganges.

9. Verfahren nach Anspruch 7 oder 8,
**dadurch gekennzeichnet,**
**dass** der Antrieb (7) einen Hydraulikzylinder (9) aufweist, der so an eine der zweiten Platte (4) vorgelagerte und mit der Schiene (2) verbundene dritte Platte (8) aufgesteckt wird, dass sein verschieblicher Kolben (11) den notwendigen Spanndruck auf die zweite Platte (4) ausübt und die Filterkassetten (19) verspannt.

10. Verfahren nach einem der Ansprüche 7 bis 9,
**dadurch gekennzeichnet,**
**dass** zum Autoklavieren der Antrieb (7) aufgesteckt und eine dem Zugstangendruck entsprechender reduzierter Spanndruck auf die zweite Platte (4) ausgeübt wird, dass die Zugstange (5) angezogen und die Platten (3, 4) über das Federspannelement (6) mit dem vorgesehenen Zugstangendruck verspannt werden,
**dass** der Antrieb (7) entspannt und abgenommen wird, dass nach dem Autoklavieren der Antrieb (7) wieder aufgesteckt und der für den Filtrationsprozess vorgesehene Spanndruck eingestellt wird, und dass die Zugstange (5) entlastet wird.

11. Verfahren nach einem der Ansprüche 7 bis 10,
**dadurch gekennzeichnet,**
**dass** der Filterhalter (1) mit einem an den Filterkassetten (19) angeschlossenen Schlauchset autoklaviert und anschließend aseptisch in einen bereits sterilen Prozessablauf eingebunden wird.

## Claims

1. Filter holder (1) for receiving filter cassettes (19), having two plates (3, 4) which are movable relative to one another on at least one rail (2) and between which the filter cassettes can be clamped, and having a drive (7), wherein a first plate (3) is fixedly arranged and a second parallel plate (4) is arranged above the drive (7) such that it can be slid with a predefinable tension pressure, and wherein the drive (7) is designed as a hydraulic cylinder (9) on which pressure can be applied, by means of the movable piston (11) of which the second plate (4) can be clamped against the filter cassettes (19) with the pre-set tension pressure, **characterized in that** the drive (7) is designed to be removable, that there is provided at least one tension rod (5) with a spring clamping element (6) that is parallel to the at least one rail (2) by means of which the two plates (3, 4) can be connected to one another and clamped with a predefinable tension rod pressure against the filter cassettes (19).

2. The filter holder according to Claim 1, **characterized in that** a third plate (8) that is fixedly connected to the at least one rail (2) is arranged offset from the second plate (4) on the side of the latter facing away from the first plate (3), and that the removable drive (7) can be mounted on the third plate (8).

3. The filter holder according to Claim 1 or 2, **characterized in that** the hydraulic cylinder (9) is connected to the pump (15) by means of a hydraulic line (14) and that a pressure gauge (16) is arranged between the pump (15) and the hydraulic cylinder (9).

4. The filter holder according to any of Claims 1 through 3, **characterized in that** four rails (2) are provided on which the second plate (4) is movable relative to the first plate (3).

5. The filter holder according to any of Claims 1 through 4, **characterized in that** two tension rods (5) each having one spring tensioning element (6) are provided and that by means of said rods the two plates (3, 4) can be connected to each other and can be clamped against the filter cassettes (19) with the predefinable tension rod pressure.

6. The filter holder according to any of Claims 1 through 5, **characterized in that** without the drive (7) the filter holder (1) is autoclavable with the filter cassettes (19), which are pretensioned by means of the at least one tensioning rod (5) having a spring tensioning element (6).

7. A method for sterilizing a filter holder (1) in an autoclave with filter cassettes (19), the latter being clamped between two plates (3, 4) which are movable relative to each other on at least one rail (2), wherein for sterilizing purposes, the second movable plate (4) is clamped by means of at least one tensioning rod (5), which has a spring tensioning element (6) and is arranged parallel to the rail (2), against the first plate (3) which is fixedly connected to the rail, and the filter holder (1) is autoclaved in the autoclave, **characterized in that** after autoclaving, a drive (7) is fixedly connected to the rail (2) for the filtration process, and that the drive (7) clamps the second plate (4) against the first plate (3) with a predefined clamping pressure, and that the tensioning rod (5) is released by means of the spring tensioning element (6).

8. The method according to Claim 7, **characterized in that** the tension rod pressure applied to the clamping plates (3, 4) for sterilizing purposes is lower than the clamping pressure for the clamping plates (3, 4) during the filtration process.

9. The method according to Claim 7 or 8, **characterized in that** drive (7) has a hydraulic cylinder (9) which is mounted onto third plate (8), which is positioned offset from the second plate (4) and is connected to the rail (2), in such a manner that its movable piston (11) applies the necessary clamping pressure on the second plate (4) and clamps the filter cassettes (19).

10. The method according to any of Claims 7 through 9, **characterized in that** for autoclaving, the drive (7) is mounted and a reduced clamping pressure, which corresponds to the tension rod pressure, is applied to the second plate (4), and that the tension rod (5) is tightened and the plates (3, 4) are clamped with the predetermined tension rod pressure by means of the spring tensioning element (6), and that the drive (7) is released and removed, and that after autoclaving, the drive (7) is once again mounted and the tensioning pressure predetermined for the filtration process is set, and that the tension rod (5) is released.

11. The method according to any of Claims 7 through 10, **characterized in that** the filter holder (1) is autoclaved with a hose set connected to the filter cassettes (19) and then aseptically integrated into an already sterile process workflow.

## Revendications

1. Porte-filtre (1) destiné à recevoir des cassettes filtrantes (19), avec deux plaques (3, 4) qui sont mobiles l'une par rapport à l'autre sur au moins un rail (2) et entre lesquelles les cassettes filtrantes (19) peuvent être serrées, et avec un mécanisme d'entraînement (7), sachant qu'une première plaque (3) est fixe et qu'une deuxième plaque (4), parallèle, peut être déplacée au moyen du mécanisme d'entraînement (7) avec une force de serrage prédéfinissable, et sachant que le mécanisme d'entraînement (7) est formé par un vérin hydraulique (9) qui peut être sollicité en pression et au moyen du piston mobile (11) duquel la deuxième plaque (4) peut être serrée avec la force de serrage prédéfinissable contre les cassettes filtrantes (19),
**caractérisé en ce que**
le mécanisme d'entraînement (7) est conçu démontable et **en ce qu'**il est prévu, parallèlement au rail (2) au moins unique, au moins un tirant (5) muni d'un élément de serrage à ressort (6), au moyen duquel les deux plaques (3, 4) peuvent être reliées entre elles et serrées avec une force de tirant prédéfinissable contre les cassettes filtrantes (19).

2. Porte-filtre selon la revendication 1, **caractérisé en ce que** la deuxième plaque (4) est précédée, sur son côté opposé à la première plaque (3), d'une troisième plaque (8) fixement reliée au rail (2) au moins unique, et **en ce que** le mécanisme d'entraînement démontable (7) peut être fixé sur la troisième plaque (8).

3. Porte-filtre selon la revendication 1 ou 2, **caractérisé en ce que** le vérin hydraulique (9) est relié à une pompe (15) par l'intermédiaire d'une conduite hydraulique (14), et **en ce qu'**un manomètre (16) est disposé entre la pompe (15) et le vérin hydraulique (9).

4. Porte-filtre selon l'une des revendications 1 à 3, **caractérisé en ce qu'**il est prévu quatre rails (2), sur lesquels la deuxième plaque (4) est mobile par rapport à la première plaque (3).

5. Porte-filtre selon l'une des revendications 1 à 4, **caractérisé en ce qu'**il est prévu deux tirants (5) munis chacun d'un élément de serrage à ressort (6), au moyen desquels les deux plaques (3, 4) peuvent être reliées entre elles et serrées avec la force de tirant prédéfinissable contre les cassettes filtrantes (19).

6. Porte-filtre selon l'une des revendications 1 à 5, **caractérisé en ce que** le porte-filtre (1) peut être passé à l'autoclave sans le mécanisme d'entraînement (7), avec les cassettes filtrantes (19) qui sont précontraintes au moyen du tirant (5) au moins unique muni de l'élément de serrage à ressort (6).

7. Procédé pour stériliser un porte-filtre (1) dans un autoclave avec des cassettes filtrantes (19) qui sont serrées entre deux plaques (3,4) mobiles l'une par rapport à l'autre sur au moins un rail (2), sachant que, pour la stérilisation, la deuxième plaque mobile (4) peut, au moyen d'au moins un tirant (5) disposé parallèlement au rail (2) et muni d'un élément de serrage à ressort (6), être serrée avec une force de tirant prédéfinie contre la première plaque (3) fixement reliée au rail (2), et que le porte-filtre (1) est passé à l'autoclave, **caractérisé en ce que**, à la suite du passage à l'autoclave, un mécanisme d'entraînement (7) est relié à poste fixe au rail (2) pour le processus de filtration, **en ce que** le mécanisme d'entraînement (7) serre la deuxième plaque (4) contre la première plaque (3) avec une force de serrage prédéfinie, et **en ce qu'**on relâche le tirant (5) muni de l'élément de serrage à ressort (6).

8. Procédé selon la revendication 7, **caractérisé en ce que** la force de tirant pour serrer les plaques (3, 4) pour la stérilisation est inférieure à la force de serrage pour serrer les plaques (3, 4) pendant l'opération de filtration.

9. Procédé selon la revendication 7 ou 8, **caractérisé en ce que** le mécanisme d'entraînement (7) présente un vérin hydraulique (9) qui est monté sur une troisième plaque (8), précédant la deuxième plaque (4) et reliée au rail (2), de telle sorte que son piston mobile (11) exerce la force de serrage nécessaire sur la deuxième plaque (4) et serre les cassettes filtrantes (19).

10. Procédé selon l'une des revendications 7 à 9, **caractérisé en ce que**, pour le passage à l'autoclave, on monte le mécanisme d'entraînement (7) et on exerce sur la deuxième plaque (4) une force de serrage réduite correspondant à la force de tirant, **en ce qu'**on serre le tirant (5) et que les plaques (3, 4) sont, au moyen de l'élément de serrage à ressort (6), serrées avec la force de tirant prévue, **en ce qu'**on relâche et démonte le mécanisme d'entraînement (7), **en ce que**, à la suite du passage à l'autoclave, on remonte le mécanisme d'entraînement (7) et qu'on règle la force de serrage prévue pour le processus de filtration, et **en ce qu'**on relâche le tirant (5).

11. Procédé selon l'une des revendications 7 à 10, **caractérisé en ce que** le porte-filtre (1) est passé à l'autoclave avec un jeu de tuyaux souples raccordé aux cassettes filtrantes (19), puis est incorporé de manière aseptique dans un processus déjà stérile.
